# EUROPÄISCHE PATENTSCHRIFT

(11) **EP 0 405 393 B1**
(45) Veröffentlichungstag und Bekanntmachung des Hinweises auf die Patenterteilung: **29.09.1993**
(21) Anmeldenummer: 90112029.5
(22) Anmeldetag: 25.06.1990
(51) Int. Cl.: B65D 57/00, A61F 13/02, B65D 75/52

(54) **Vorrichtung zur Verpackung von haftklebenden Substratabschnitten und ihre Verwendung**
Device for packaging self-adhesive substrate portions and its application
Dispositif pour emballer des portions de substrat auto-adhésives et leur utilisation

(30) Priorität: 30.06.1989 DE 3921434
(43) Veröffentlichungstag der Anmeldung: 02.01.1991
(73) Patentinhaber: LTS Lohmann Therapie-Systeme GmbH & Co. KG, 56567 Neuwied (DE)
(72) Erfinder: Anhäuser, Dieter, D-5451 Melsbach (DE); Huhn, Ralf, Dipl.-Ing., D-5330 Königswinter 41 (DE)
(74) Vertreter: Flaccus, Rolf-Dieter, Dr.

(56) Entgegenhaltungen:
- WO-A-89/04158
- DE-A- 1 936 607
- US-A- 2 577 945
- US-A- 3 068 860
- US-A- 4 498 586

## Beschreibung

Die Erfindung betrifft eine Vorrichtung nach dem Oberbegriff des Patentanspruchs 1 sowie deren Verwendung.

Die Herstellung und Verwendung flächiger haftklebender Substratabschnitte ist bekannt. Dabei ergibt sich oft die Notwendigkeit, die Abschnitte für den Zeitraum zwischen Herstellung und Anwendung vor dem Verdampfen von flüchtigen Bestandteilen zu schützen. Dies kann dadurch bewerkstelligt werden, daß die haftklebenden Substratabschnitte nach Schutz ihrer haftklebenden Fläche durch eine wieder ablösbare Trägerschicht in einen sie allseitig umschließenden Beutel eingesiegelt werden. In den meisten Fällen überragt dabei die Trägerschicht die haftklebende Fläche, um den Abzug der Trägerschicht vor Gebrauch zu erleichtern. Der Nachteil dieser Konstruktion liegt aber darin, daß während der Lagerzeit dieser so verpackten Substratabschnitte durch den kalten Fluß des Haftklebers um den Substratabschnitt herum Haftkleber austritt, der eine Verklebung mit den Verpackungsflächen verursacht, so daß die Entnahme des Laminates bei nur einseitig geöffneter Packung erschwert oder gar unmöglich wird.

Aus der DE-A-1 936 607 ist ein Wundschnellverband bekannt. Dieser besteht aus einem mit einer Haftklebeschicht versehenen Trägerstreifen, einer darauf angebrachten Wundauflage zur unmittelbaren Abdeckung der Wunde in Form eines kissenförmigen Gebildes aus Mull, Zellstoff, Faservlies oder ähnlichem Material und einer darüber angeordneten Schutzabdeckung. Diese ist als flächiges Einstück ausgebildet, das entlang einer vorbestimmten Linie geteilt werden kann. Diese Teilungslinie ist durch eine Schwächung des Materials wie Perforierung bzw. Einschnitte gebildet.

Es ist Aufgabe der vorliegenden Erfindung, eine einfache Vorrichtung zur Erhöhung der Lagerstabilität von in Beuteln verpackten, haftklebend ausgerüsteten flächigen Substratabschnitten zu schaffen, deren Haftklebefläche durch eine diese mindestens zum Teil überragende Trägerschicht geschützt ist. Hierdurch soll insbesondere verhindert werden, daß es durch Wanderung des Haftklebers aufgrund von kaltem Fluß zu einer Verklebung der Trägerschicht mit der Verpackung kommt.

Die Lösung der Aufgabe wird überraschenderweise darin gefunden, daß in dem die Haftklebefläche überragenden Teil der Trägerschicht auf der Trägerschicht fixierte oder aus der Trägerschicht selbst geformte Erhebungen angebracht sind, die auf der Seite der Trägerschicht angeordnet sind, auf der auch der Substratabschnitt angebracht ist, als Abstandshalter zwischen Trägerschicht und Beutel.

Die Erfindung schließt zwei prinzipielle Möglichkeiten ein. Einmal können die Abstandshalter durch Hinzufügen von Fremdmaterialien oder zum anderen durch Ausnutzen der Trägerschicht selbst erzeugt werden. In ersten Fall werden die Abstandshalter durch Aufbringen von Heiß-Schmelz-Polymeren gebildet, die nach Abkühlen fest auf der Trägerschicht verankert sind, oder es werden nichtklebende Materialien durch Permanentkleber auf der Trägerschicht fixiert. Im zweiten Falle werden durch Knick-, Präge- oder Tiefziehvorgänge die überstehenden Trägermaterialflächen so verformt, daß in Richtung der dem Substratabschnitt benachbarten Verpackungsfläche Erhebungen entstehen, die als Abstandshalter wirken. Dies kann bspw. erreicht werden durch Umknicken von Ecken der Tägerschicht oder auch durch Knicken von durch Einschneiden der Selten der Trägerschicht erhaltenen Laschen. Eine weitere Möglichkeit bietet das Prägen oder Tiefziehen von Noppen im überstehenden Teil der Trägerschicht, die in Richtung der dem Substratabschnitt benachbarten Verpackungsfläche gerichtet sind. Auch Stege, die aus dem überstehenden Teil der Trägerschicht geprägt oder tiefgezogen sind und Ecken des Substratabschnittes umwinkeln oder zusammenhängend parallel zu den Rändern des Substratabschnittes verlaufen, vermögen die Aufgabe als Abstandshalter zu erfüllen. Natürlich gelten diese Ausführungen auch für Mehrfachanordnungen von Substratabschnitten auf der Trägerschicht.

Das die Trägerschicht bildende Trägermaterial kann auch mehrschichtig sein und muß zur haftklebenden Substratabschnittsfläche hin so ausgerüstet sein, daß die Wiederablöin denen die Abstandshalter aus der Trägerschicht selbst gefornt werden, muß diese warm oder kalt bleibend verformbar sein. Als geeignete Materialien hierfür seien beispielsweise thermoplastische Polymere, Metalle oder Kombinationen beider genannt.

Die Bildung der Abstandshalter kann vor oder nach dem Aufbringen des Substratabschnittes beliebiger Kontur auf die Trägerschicht erfolgen. Ihre Höhe wird im wesentlichen durch die Dicke des Substratabschnittes bestimmt.

Die erfindungsgemäße Vorrichtung wird besonders zur Verpakkung von Pflastern, transdermalen therapeutischen Systemen oder dgl. angewandt.

Die Erfindung wird anhand der folgenden Figuren beispielhaft weiter erläutert. Die Figuren 1-5 erläutern die Erfindung ohne Darstellung der Verpackung. So zeigt
Fig. 1 die perspektivische Darstellung einer erfindungsgemäßen Ausführung,
Fig. 2 - 5 die perspektivischen Darstellungen weiterer Ausführungen der Erfindung,
Fig. 6 den nicht maßstabsgerechten Querschnitt durch einen verpackten Substratabschnitt gemäß der Erfindung.

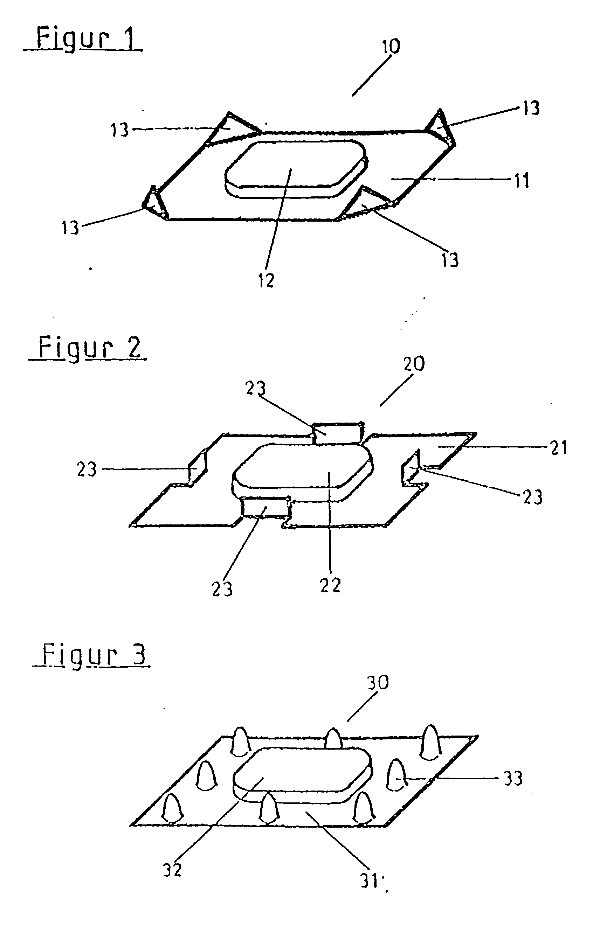

In Figur 1 ist eine perspektivische Darstellung 10 einer erfindungsgemäßen Ausführungsform wiedergegeben, bei der auf einer Trägerschicht 11 ein substratabschnitt 12 angeordnet ist. Die Ecken 13 des überstehenden Teils der Trägerschicht sind nach oben gebogen und wirken als Abstandshalter.

Die Figur 2 zeigt eine erfindungsgemäße Ausführungsform 20, bei der durch Einschnitte in die Seiten der den Substratabschnitt 22 überragenden Trägermaterialflächen 21 Laschen 23 gebildet sind, die durch Aufbiegen die Abstandshaltung übernehmen.

Die Anordnung 30 in Fig. 3 zeigt aus dem den Substratabschnitt 32 überragenden Teil 31 der Trägerschicht geformte Noppen 33, die als Abstandshalter wirken.

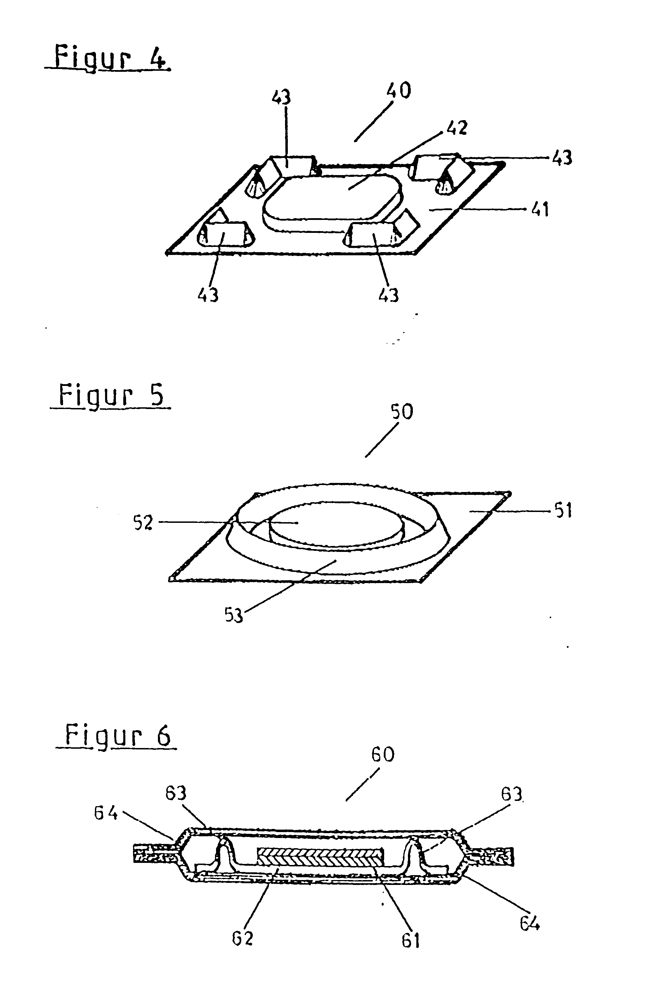

Bei der Anordnung 40 in Fig. 4 sind aus dem den Substratabschnitt 42 überragenden Teil 41 der Trägerschicht die Ecken des Substratabschnittes umwinkelnde Stege gezeigt, die als Abstandshalter fungieren.

Figur 5 zeigt schließlich eine Anordnung 50, bei der durch Prägung oder Tiefziehen des überstehenden Teiles 51 der Trägerschicht ein parallel zur Kante des Substratabschnittes 52 gebildeter Steg 53 die Abstandshaltung besorgt. In Figur 6 ist ein Querschnitt 60 durch einen verpackten Substratabschnitt 61 gezeigt, wobei die Verpackungsfläche 64 durch die Abstandshaltung 63 von der Trägerschicht 62 ferngehalten wird.

## Patentansprüche

1. Vorrichtung (10, 20, 30, 40, 50, 60) zur Erhöhung der Lagerstabilität von in Beuteln verpackten, haftklebend ausgerüsteten flächigen Substratabschnitten (12, deren Haftklebefläche durch eine diese mindestens zum Teil überragende Trägerschicht (11) geschützt ist, dadurch gekennzeichnet, daß in dem die Haftklebefläche überragenden Teil der Trägerschicht (11, 21, 31, 41, 51, 62) auf der Trägerschicht fixierte oder aus der Trägerschicht selbst geformten Erhebungen (13, 23, 33, 43, 53, 63) angebracht sind, die auf der Seite der Trägerschicht angeordnet sind, auf der auch der Substratabschnitt angebracht ist, als Abstandshalter zwischen Trägerschicht und Beutel.

2. Vorrichtung (10, 20, 30, 40, 50, 60) nach Anspruch 1, dadurch gekennzeichnet, daß die Abstandshalter (13, 23, 33, 43, 53) aus Heiß-Schmelz-Polymeren bestehen, die bei Zimmertemperatur keine Klebrigkeit besitzen.

3. Vorrichtung (10) nach Anspruch 1, dadurch gekennzeichnet, daß die Abstandshalter (13) aus nichtklebenden Materialien gebildet sind, die mit Permanentklebern auf der Trägerschicht (11) fixiert sind.

4. Vorrichtung (10) nach Anspruch 1, dadurch gekennzeichnet, daß die Abstandshalter (13) durch Umknicken der Ecken (13) der überstehenden Trägerschicht (11) in Richtung der dem Substratabschnitt (12) benachbarten Verpackungsfläche gebildet sind.

5. Vorrichtung (20) nach Anspruch 1, dadurch gekennzeichnet, daß die Abstandshalter (23) durch Umknicken von durch Einschneiden der Ränder der überstehenden Trägerschicht (21) erhaltenen Laschen (23) in Richtung der dem Substratabschnitt (22) benachbarten Verpackungsfläche gebildet sind.

6. Vorrichtung (30) nach Anspruch 1, dadurch gekennzeichnet, daß die Abstandshalter (33) durch Prägung oder Tiefziehen von Noppen (33) im überstehenden Teil der Trägerschicht (31) in Richtung der dem Substratabschnitt benachbarten Verpackungsfläche gebildet sind.

7. Vorrichtung (40) nach Anspruch 1, dadurch gekennzeichnet, daß die Abstandshalter (43) durch Prägung oder Tiefziehen die Ecken des Substratabschnitts umwinkelnden Stege (43) im überstehenden Teil der Trägerschicht (41) in Richtung der dem Substratabschnitt (42) benachbarten Verpackungsfläche gebildet sind.

8. Vorrichtung (50) nach Anspruch 1, dadurch gekennzeichnet, daß die Abstandshalter (53) durch Prägung oder Tiefziehen von zusammenhängenden Stegen (53) parallel zu den Rändern des Substratabschnitts (52) im überstehenden Teil der Trägerschicht (51) in Richtung der dem Substratabschnitt (52) benachbarten Verpakkungsfläche gebildet sind.

9. Vorrichtung (10, 20, 30, 40, 50, 60) nach einem der vorangehenden Ansprüche, dadurch gekennzeichnet, daß sich Mehrfachanordnungen der Substratabschnitte auf der Trägerschicht befinden.

10. Vorrichtung (10, 20, 30, 40, 50, 60) nach einem der vorangehenden Ansprüche, dadurch gekennzeichnet, daß das die Trägerschicht (11, 21, 31, 41, 51, 62) bildende Trägermaterial mehrschichtig ist.

11. Vorrichtung (10, 20, 30, 40, 50, 60) nach Ansprüchen 4 bis 10, dadurch gekennzeichnet, daß das die Trägerschicht (11, 21, 31 41, 51, 62) bildende Trägermaterial warm oder kalt bleibend verformbar ist.

12. Verwendung der Vorrichtung nach einem der vorangehenden Ansprüche zur Verpackung von Pflastern, transdermalen therapeutischen Systemen oder dgl..

## Claims

1. Device (10,20,30,40,50,60) for increasing the storage stability of sheet-like substrate sections (12) being packed in bags and rendered pressure-sensitive adhesive, the pressure-sensitive surface of which being protected by a supporting layer (11) projecting beyond said pressure-- sensitive adhesive surface at least partially, characterized in that elevations (13,23,33,43,53,63) are fixed on the supporting layer (11,21,31,41,51,62) or are formed from the supporting layer itself and are located on that side of the supporting layer on which the substrate section is applied, which elevations are positioned as spacers between the supporting layer and the packing.

2. A device (10,20,30,40,50,60) according to claim 1, characterized in that the spacers (13,23,33,43,53) consist of hot-melt-polymers which are not tacky at room temperature.

3. A device (10) according to claim 1, characterized in that the spacers (13) are formed from non-sticking materials which are fixed on the supporting layer (11) by means of a permanent adhesive.

4. A device (10) according to claim 1, characterized in that the spacers (13) are formed by folding the edges (13) of the projecting carrier layer (11) in the direction of the packing surface adjacent to the substrate section (12).

5. A device (20) according to claim 1, characterized in that the spacers (23) are formed by folding the tabs (23) in the direction of the packing surface adjacent to the substrate section (22), said tabs being obtained by cutting-in the borders of the projecting supporting layer (21).

6. A device (30) according to claim 1, characterized in that the spacers (33) are formed by embossing or deep-- drawing knobs (33) within the projecting portion of the carrier layer (31) in the direction of the packing surface adjacent to the substrate section.

7. A device (40) according to claim 1, characterized in that the spacers (43) are formed by embossing or deep-- drawing webs (43) in the projecting part of the supporting layer (41) in the direction of the packing surface being adjacent to the substrate section (42), said webs (43) forming angles about the edges of the substrate section.

8. A device (50) according to claim 1, characterized in that the spacers (53) are created by embossing or deep-- drawing of coherent webs (53), which run parallel to the borders of the substrate section (52), in the projecting part of the supporting layer (51) in the direction of the packing surface adjacent to the substrate section.

9. A device (10,20,30,40,50,60) according to any one of the preceding claims, characterized in that multiple arrangements of the substrate sections are positioned on the carrier layer.

10. A device (10,20,30,40,50,60) according to any one of the preceding claims, characterized in that the support material forming the supporting layer (11,21,31,41,51,62) is multi-layered.

11. A device (10,20,30,40,50,60) according to claims 4 to 10, characterized in that the support material forming the supporting layer (11,21,31,41,51,62) is of permanent hot or cold deformability.

12. The use of the device as defined in any one of the preceding claims for the packaging of plasters, transdermal therapeutic systems, and the like.

## Revendications

1. Dispositif (10, 20, 30, 40, 50, 60) en vue d'accroître la stabilité de conservation de pièces de substrat (12) plates dotées d'une colle et emballées en poches, dont la surface adhérente est protégée par une couche porteuse (11) au moins partiellement débordante, caractérisé en ce que dans la partie de la couche porteuse (11, 21, 31, 41, 51, 62) débordant de la surface adhérente sont disposées des saillies (13, 23, 33, 43, 53, 63) fixées sur la couche porteuse ou formées à partir de la couche porteuse elle-même, ces saillies étant pratiquées sur la face de la couche porteuse sur laquelle la pièce de substrat est placée, et servant d'écarteurs entre la couche porteuse et la poche.

2. Dispositif (10, 20, 30, 40, 50, 60) selon la revendication 1, caractérisé en ce que les écarteurs (13, 23, 33, 43, 53) sont constitués de polymères thermoplastiques qui ne possèdent aucune capacité d'adhérence à température ambiante.

3. Dispositif (10) selon la revendication 1, caractérisé en ce que les écarteurs (13) sont formés en un matériau non adhérent, et sont fixés sur la couche porteuse (11) à l'aide de colles permanentes.

4. Dispositif (10) selon la revendication 1, caractérisé en ce que les écarteurs (13) sont formés par repliage des coins (13) de la couche porteuse (11) débordante, en direction de la surface de l'emballage voisine de la pièce de substrat (12).

5. Dispositif (20) selon la revendication 1, caractérisé en ce que les écarteurs (23) sont formés par pliage, en direction de la surface de l'emballage voisine de la pièce de substrat (22), de languettes (23) obtenues par découpage des bords de la couche porteuse (21) débordante.

6. Dispositif (30) selon la revendication 1, caractérisé en ce que les écarteurs (33) sont formés par estampage ou emboutissage de saillies (33) dans la partie débordante de la couche porteuse (31), en direction de la surface de l'emballage voisine de la pièce de substrat.

7. Dispositif (40) selon la revendication 1, caractérisé en ce que les écarteurs (43) sont formés par estampage ou emboutissage de saillies (43) entourant les coins de la pièce de substrat, dans la partie débordante de la couche porteuse (41), en direction de la surface de l'emballage voisine de la pièce de substrat (42).

8. Dispositif (50) selon la revendication 1, caractérisé en ce que les écarteurs (53) sont formés par estampage ou emboutissage de saillies (53) continues s'étendant parallèlement aux bords de la pièce de substrat (52) dans la partie débordante de la couche porteuse (51), en direction de la surface de l'emballage voisine de la pièce de substrat (52).

9. Dispositif (10, 20, 30, 40, 50, 60) selon l'une des revendications précédentes, caractérisé en ce que plusieurs pièces de substrat sont placées sur la couche porteuse.

10. Dispositif (10, 20, 30, 40, 50, 60) selon l'une des revendications précédentes, caractérisé en ce que le matériau porteur formant la couche porteuse (11, 21, 31, 41, 51, 62) est constitué de plusieurs couches.

11. Dispositif (10, 20, 30, 40, 50, 60) selon les revendications 4 à 10, caractérisé en ce que le matériau porteur formant la couche porteuse (11, 21, 31, 41, 51, 62) est déformable de manière permanente, à chaud ou à froid.

12. Utilisation du dispositif selon l'une des revendications précédentes pour l'emballage de pansements, de systèmes thérapeutiques transdermiques ou similaires.
